(19) Europäisches Patentamt
European Patent Office
Office européen des brevets

(11) Veröffentlichungsnummer: **0 206 021**
**B1**

(12) # EUROPÄISCHE PATENTSCHRIFT

(45) Veröffentlichungstag der Patentschrift: **10.08.88**

(21) Anmeldenummer: **86107568.7**

(22) Anmeldetag: **04.06.86**

(51) Int. Cl.⁴: **C 07 C 39/367,** C 07 C 69/16, C 07 C 69/63, C 07 C 125/04, C 07 F 9/40 // A61K31/055, A61K31/22

(54) **Neue Derivate des 1,1,2,2-Tetramethyl-1,2-bis-(2-fluor-4-hydroxyphenyl)-ethans.**

(30) Priorität: **08.06.85 DE 3520622**

(43) Veröffentlichungstag der Anmeldung: **30.12.86 Patentblatt 86/52**

(45) Bekanntmachung des Hinweises auf die Patenterteilung: **10.08.88 Patentblatt 88/32**

(84) Benannte Vertragsstaaten: **AT BE CH DE FR GB IT LI LU NL SE**

(56) Entgegenhaltungen:
**DE-A-2 927 050**

**JOURNAL OF MEDICINAL CHEMISTRY, Band 28, Nr. 9, September 1985, Seiten 1295-1301, American Chemical Society; R.W. HARTMANN et al.: "Ring-substituted 1,1,2,2-tetraalkylated 1,2-bis(hydroxyphenyl)ethanes. 4. Synthesis, estrogen receptor binding affinity, and evaluation of antiestrogenic and mammary tumor inhibiting activity of symmetrically disubstituted 1,1,2,2-tetramethyl-1,2-bis(hydroxyphenyl)ethanes"**

(73) Patentinhaber: **ASTA Pharma AG, Weismüllerstrasse 45, D-6000 Frankfurt am Main 1 (DE)**

(72) Erfinder: **Schönenberger, Helmut, Prof. Dr., Ahornstrasse 14, D-8401 Pentling (DE)**
Erfinder: **Hartmann, Rolf W., Dr., Am Mühlberg 3, D-8403 Bad Abbach (DE)**
Erfinder: **Schneider, Martin, Dr., Steinmetzstrasse 11, D-8400 Regensburg (DE)**
Erfinder: **Schwarz, Walter, Hauptstrasse 29, D-8401 Pentling (DE)**
Erfinder: **Engel, Jürgen, Dr., Erlenweg 3, D-8755 Alzenau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1988

**Beschreibung**

Das echte Antiöstrogen 1,1,2,2-Tetramethyl-1,2-bis(4-hydroxyphenyl)ethane (R.W. Hartmann, Eur. J. Cancer Clin. Oncol 1983, 19, 959) weist eine beträchtliche Affinität zum Östrogenrezeptor auf (relative Bindungsaffinität = 3,6). Diese Verbindung zeigt beispielsweise eine starke Wirkung als Antiöstrogen durch Hemmung des durch Östron beziehungsweise Östradiol stimulieren Uteruswachstums juveniler Mäuse und hemmt das Wachstum des durch 7,12-Dimethyl-benzanthracen (DMBA) induzierten Mammacarcinoms der Ratte (Sprague Dawley Ratte), einem experimentellen Tumor, der in seinem Verhalten dem menschlichen Mammacarcinom (MC) sehr ähnlich ist.

Die erfindungsgemäßen Verbindungen weisen überraschend eine erheblich gesteigerte Affinität zum Östrogenrezeptor auf (relative Bindungsaffinität der Verbindung gemäß Beispiel 1 = 11) und erweisen sich am DMBA-Tumor als wesentlich stärker wirksam als die bekannte Substanz.

Die erfindungsgemäßen Substanzen sind Antiöstrogene und zeigen zum Beispiel im Uterus-Gewichts-Testen an der juvenilen Maus eine stark östrogen-antagonistische Wirkung.

Die erfindungsgemäßen Verbindungen besitzen eine starke Antitumorwirkung an östrogenrezeptor-positiven Tumoren, da sie sich in hormonabhängigen Tumoren anreichern.

Insbesondere eignen sich die erfindungsgemäßen Verbindungen zur Behandlung von hormonabhängigen Tumoren, insbesondere des hormonabhängigen Mammacarcinoms; sie sind aber auch zum Beispiel für die Behandlung des Prostatacarcinoms geeignet, da sie bei der Maus das Gewicht von Targetorganen (wie zum Beispiel Samenblase) erniedrigen und eine starke Absenkung des Testosteronspiegels verursachen. Von großer Bedeutung ist auch, daß die erfindungsgemäßen Verbindungen nur geringfügige östrogene Effekte zeigen beziehungsweise sogar antiöstrogen wirken. Bei den Verbindungen der Formel I, worin $R_1$ und/oder $R_2$ keine Wasserstoffatome sind, unterbleibt die Phase der Metabolisierung zum wasserlöslichen Metaboliten. Die Halbwertszeit dieser Verbindungen ist daher erhöht und die Antitumorwirkung daher langanhaltend. Solche Verbindungen sind daher insbesondere für eine perorale Verabreichung oder für eine Applikation als Depotform geeignet. Die Verbindung der Formel I, worin die Reste $R_1$, und $R_2$ die Gruppe $-PO(OH)_2$ darstellen, ist als wasserlösliche Verbindung insbesondere für die intravenöse Applikation geeignet.

Vorzugsweise sind die Reste $R_1$ und $R_2$ gleich. Falls einer oder beide Substituenten $R_1$ und $R_2$ Alkylreste enthalten beziehungsweise Alkanoylreste, Halogenalkanoylreste oder Alkenoylreste darstellen, können diese gerade oder verzweigt sein. Falls die Reste $R_1$ und/oder $R_2$ $C_2$-$C_8$-Alkanoylgruppen bedeuten, bestehen diese vorzugsweise aus 1-4 C-Atomen Insbesondere handelt es sich um die Acetylgruppe. Falls solche Alkanoylgruppen Halogenatome enthalten, können die Halogenatome sich an einem oder verschiedenen C-Atomen befinden, wobei die Zahl der Halogenatome (insbesondere Chlor und Brom) 1-3 sein kann. Beispiele für Halogenalkanoylreste sind: $CHCl_2$-CO-, $CH_2Cl$-CO, $CCl_3$-CO-, $CH_2Cl$-$CH_2$-CO, $CH_2Br$-$CH_2$-CO, $CHCl_2$-$CH_2$-CO-, $CHBr_2$-$CH_2$-CO-, $CH_2Cl$-CHCl-CO-, $CH_2Br$-CHBr-CO-, $CHCl_2$-CHCl-CO-, $CH_2Br$-CHBr-CO-,

Falls $R_1$ und/oder $R_2$ $C_3$-$C_8$-Alkenoylgruppen bedeuten, bestehen diese insbesondere aus 3 bis 6 C-Atomen. Vorzugsweise handelt es sich in diesem Falle um den Rest $CH_2$=CH-CO- (Acryloylrest).

Zu dem Verfahren:

Das erfindungsgemäße Verfahren findet in einem Temperaturbereich zwischen -70° C und +250° C statt.

Die Abspaltung der $C_1$-$C_4$-Alkylgruppen (Ethergruppen) erfolgt beispielsweise ohne Lösungsmittel oder in einem inerten Lösungsmittel mit Bortribromid, Bortrifluorid, Aluminiumchlorid, Siliziumtetrachlorid, Aluminiumtribromid, Natriumethylthiolat, $(CH_3)_3SiCl$ + NaJ bei Temperaturen zwischen -70° C und 200° C. Als Lösungsmittel für diese Etherspaltung kommen beispielsweise in Frage: aliphatische Halogenkohlenwasserstoffe wie zum Beispiel Methylenchlorid, aromatische Kohlenwasserstoffe wie Benzol, Toluol, Xylol, halogenierte aromatische Kohlenwasserstoffe wie Chlorbenzol, Dichlorbenzole, Dimethylformamid, Acetonitril, Nitrobenzol, Schwefelkohlenstoff und Dioxan.

Weiterhin kann diese Etherspaltung auch mittels konzentriertes Jodwasserstoffsäure, Bromwasserstoffsäure (insbesondere in Gemischen mit Essigsäure und Essigsäureanhydrid), Pyridinhydrochlorid, Schwefelsäure, Trifluoressigsäure,-Phosphorsäure, Chlor-, Methyl, p-Toluol-Sulfonsäure, Methylmagnesiumjodid mit oder ohne Lösungsmittel bei Temperaturen zwischen 20° C bis 250° C erfolgen. Als Lösungsmittel für die zuletzt genannte Spaltung kommen beispielsweise in Betracht: aliphatische Ether mit Alkylresten aus 1 bis 6 C-Atomen, Toluol und Benzol.

Die Acylierung gemäß dem Herstellungsverfahren erfolgt durch eine Säure der Formel R-OH, wobei R eine Aminocarbonylgrupe, eine $C_1$-$C_6$-Alkylaminocarbonylgruppe, eine Di-$C_1$-$C_6$-alkylaminocarbonylgruppe, die Gruppe $PO(OH)_2$, eine $C_2$-$C_8$-Alkanoylgruppe oder eine $C_2$-$C_8$ Halogenalkanoylgruppe ist und eine solche Säure vorzugsweise aktiviert ist. Falls für die Acylierung eine solche aktivierte Säure verwendet wird, handelt es sich vorzugsweise um Verbindungen der Formel R-X, worin R die oben angegebenen

Bedeutungen hat und X ein Halogenatom, eine Gruppe der Formel -OR', -SR' oder eine Gruppe der Formel $-OSO_3H$ oder -OCO-R'' bedeutet und R' einen $C_1$-$C_6$-Alkylrest oder im Falle von -OR' beziehungsweise -SR' auch einen Phenylrest, einen durch Nitrogruppen, $C_1$-$C_4$-Alkoxygruppen $C_1$-$C_4$-Alkylgruppen oder Halogenatome (Chlor, Fluor, Brom) substituierten Phenylrest, einen Cyanmethylrest oder einen Carboxymethylrest und R'' einen geraden oder verzweigten $C_1$-$C_7$-Alkylrest, einen $C_1$-$C_7$-Halogenalkylrest, einen $C_1$-$C_6$-Alkoxyrest, einen Phenoxyrest oder einen Benzyloxyrest darstellt, wobei R auch die Gruppe COCl, $POCl_2$ oder die Cyangruppe sein kann, falls X Halogen ist und sich im Falle von COCl eine anschließende Umsetzung in üblicher Weise mit $NH_3$, einem $C_1$-$C_6$-Alkylamin oder einem Di-$C_1$-$C_6$-alkylamin anschließt und im Falle von $POCl_2$ oder der Cyangruppe noch eine Verseifung mittels einer Mineralsäure (Schwefelsäure oder Salzsäure, beziehungsweise einer Mischung beider Säuren) anschließt. Eine solche Verseifung erfolgt zum Beispiel in wässrigem Medium bei Temperaturen zwischen 50° C und 80° C. Falls X ein Halogenatom bedeutet, handelt es sich vorzugsweise um Chlor, Brom oder Jod; falls R' beziehungsweise R'' Alkylreste, Halogenalkylreste oder Alkoxyreste bedeuten, dann sind diese vorzugsweise niedermolekular und bestehen aus 1 - 4 Kohlenstoffatomen.

Die Acylierung wird beispielsweise in einem üblichen Lösungsmittel oder Suspensionsmittel wie Wasser, gegebenenfalls unter Zusatz eines Lösungsvermittlers (zum Beispiel niedere aliphatische Alkohole, niedere aliphatische Ketone, Dimethylformamid) oder indifferenten Mitteln durchgeführt. Als Lösungsbeziehungsweise Suspensionsmittel kommen beispielsweise in Betracht: Niedrigmolekulare aliphatische Ether (zum Beispiel 4 - 10 C-Atome); niedrigmolekulare aliphatische Ketone (zum Beispiel 3 - 6 C-Atome); gesättigte cyclische Ether wie Tetrahydrofuran, Dioxan, niedrigmolekulare gesättigte Chlor- und Fluorkohlenwasserstoffe mit 1 - 5 C-Atomen, wobei die einzelnen C-Atome ein- oder mehrfach (2- bis 3-fach) durch Chlor und/oder Fluor substituiert sein können, wie Chloroform, Methylenchlorid; gegebenenfalls durch Chlor oder Brom substituierte aromatische Kohlenwasserstoffe wie Benzol, Toluol, Xylol, Chlorbenzol, Dimethylformamid, Dimethylsulfoxid, Tetramethylharnstoff, Pyridin, N-Methylpyrrolidon. Es können selbstverständlich auch Mischungen dieser genannten Lösungsmittel verwendet werden.

Die Acylierung wird zum Beispiel bei Temperaturen zwischen 0 und 200° C vorzugsweise 15 und 150° C durchgeführt.

Häufig, insbesondere wenn X ein Halogenatom oder die Gruppe -OCOR'' bedeutet, ist die Gegenwart eines säurebindenen Stoffes wie Alkalihydroxiden, Alkalicarbonaten, Alkalihydrogencarbonaten, Alkaliacetaten, Erdalkalicarbonaten, Trialkylaminen, Dialkylaminen, Pyridin und ähnlichen zweckmäßig. Dabei kann das säurebindende Agens auch gleichzeitig allein oder im Gemisch mit anderen üblichen Mitteln als Lösungsmittel benutzt werden (zum Beispiel Pyridin).

Bei der Acylierung kann man auch so vorgehen, daß man erst von der umzusetzenden Verbindung eine Alkaliverbindung herstellt, indem man sie in einem inerten Lösungsmittel wie Dioxan, Dimethylformamid, Benzol oder Toluol mit einem Alkalimetall, Alkalihydriden oder Alkaliamiden (insbesondere Natrium oder Natriumverbindungen) bei Temperaturen zwischen 0 und 150° C umsetzt und zum Beispiel dann das acylierende Agens (Verbindung R-X, X= Halogen) zufügt.

Falls die freie Säure R-OH verwendet wird, so ist deren Aktivierung durch die Gegenwart von Kondensationsmitteln wie Dicyclohexylcarbodiimid, Tetraäthylpyrophophit, 5-(3'-Sulfonphenyl)-äthylisooxazol, Schwefligsäure-bisalkylamiden (zum Beispiel $SO[N(CH_3)_2]_2$), N,N'-Carbonyldiimidazol und so weiter erforderlich (Organic Reactions, Vol. 12, 1962, Seiten 205 und 239).

Verbindungen der Formel I, worin einer oder beide Reste $R_1$ und $R_2$ eine $C_3$-$C_8$-Alkenoylgruppe darstellen, können auch aus entsprechenden Verbindungen I erhalten werden, bei denen einer oder beide dieser Reste eine $C_3$-$C_8$-Alkanoylgryppe darstellen, welche ein Chlor- oder Bromatom enthalten (vorzugsweise in β-Stellung), indem man mittels einer Base (zum Beispiel einem tertiären Amin, wie Triethylamin, Tripropylamin, N-Methylpyrrolidon und Pyridin) das Halogenatom unter Ausbildung einer Doppelbindung (zum Beispiel α-β-ständig) abspaltet. Diese Reaktion findet zum Beispiel bei Temperaturen zwischen 100 und 150° C in einem inerten Lösungsmittel (aromatische Kohlenwasserstoffe, wie Toluol, Xylol und Chlorbenzol oder Halogenwasserstoffe wie Methylenchlorid oder Chloroform) statt.

Die Acylgruppen in den Verbindungen der Formel I können solvolytisch wieder abgespalten werden, wodurch die entsprechenden freien Hydroxy-Verbindungen der Formel I erhalten werden. Diese solvolytische Abspaltung erfolgt beispielsweise durch Verseifung mit verdünnten Säuren oder mittels basischer Substanzen (Pottasche, Soda, wässrige Alkalilösungen, alkoholische Alkalilösungen, $NH_3$) bei Temperaturen zwischen 10 und 150° C, insbesondere 20 - 100° C.

Es ist auch eine selektive Abspaltung möglich, indem nur ein Acylrest ($R_1$ oder $R_2$) abgespalten wird. Eine derartige selektive Abspaltung ist beispielsweise durch Reaktion eines entsprechenden Diesters mit Alkalihydroxid im Molverhältnis 1 : 1 bei Temperaturen unter 50° C möglich.

Die Herstellung der Ausgangsverbindungen II erfolgt beispielsweise dadurch, daß man eine Verbindung der Formel

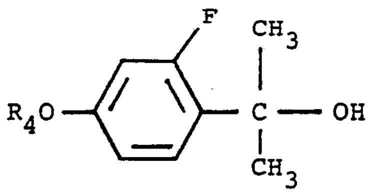

III

worin $R_4$ einen $C_1$-$C_6$-Akylrest, insbesondere den Methylrest bedeutet, in Gegenwart von Titantrichlorid und einem komplexen Alkalihydrid oder das entsprechende Chlorid (in Formel III steht dann anstelle der Hydroxygruppe Cl) mittels Ethylmagnesiumbromid in Gegenwart von Kobaltdichlorid reduktiv kuppelt.

Dieses Verfahren zur Herstellung der Ausgangsverbindungen II wird in einem Lösungs- oder Suspensionsmittel, wie gesättigen niedermolekularen aliphatischen oder cycloaliphatischen Ethern (Dimethoxyethan, Diethylether, Dioxan, Tetrahydrofuran) bei Temperaturen zwischen 30 - 100° C durchgeführt.

Die Reaktionszeit liegt beispielsweise zwischen 6 - 18 Stunden. Im Falle der reduktiven Kupplung unter Verwendung von $TiCl_3$ verfährt man beispielsweise wie folgt: Aus Titantrichlorid und einem komplexen Alkalihydrid (zum Beispiel Lithiumaluminiumhydrid) wird zuerst in einem Suspensionsmittel das Titan(II)-haltige Kupplungsreagenz nach der von Murry und Silvestri in J. Org. Chem. Band 40, Nr. 18, 1975, Seite 2687-2688 angegebenen Methode hergestellt. Als Suspensionsmittel kommen zum Beispiel in Betracht: gesättigte niedermolekulare aliphatische oder cycloaliphatische Ether wie Dimethoxyethan, Diethylether, Dioxan, Tetrahydrofuran.

Als komplexe Alkalihydride kommen zum Beispiel Alkaliborhydride oder Alkalialuminiumhydride in Frage. Als Alkaliatome kommen zum Beispiel Lithium, Natrium in Betracht.

Der Alkohol der Formel III wird dann in einem Lösungsmittel, insbesondere einem wie oben angegebenen Ether zu der Suspension des Titan(II)haltigen Kupplungsreagenzes gegeben und die so erhaltene Mischung längere Zeit auf 93° C erhitzt.

Im Falle der Verwendung von Ethylmagnesiumbromid wird beispielsweise das aus dem Alkohol III gewonnene Chlorid tropfenweise zu einer Lösung von Ethylmagnesiumbromid in einem gesättigten niedermolekularen aliphatischen cycloaliphatischen Ether (Diethylether, Dioxan, Tetrahydrofuran), der zuvor wasserfreies $COCl_2$ zugesetzt wurde, zugegeben. Diese Reaktion wird vorzugsweise bei Raumtemperatur durchgeführt (siehe hierzu J. Med. Chem. 1981, Band 24, Seite 1192-1197).

Die erfindungsgemäßen Verbindungen zeigen am DMBA-induzierten Mammacarcinom der Sprague-Dawley (SD) Ratte, am MXT-Mammacarcinom der $BD2F_1$-Maus oder am hormonabhängigen Prostatacarcinom (Dunning R 3327H) eine gute tumorhemmende Wirkung.

Beispielsweise wird am DMBA-induzierten Mammacarcinom der Sprague-Dawley Ratte bei einer Dosis von 5 mg/kg Körpergewicht Ratte eine vollständige Remission bei 74 % der Tumore erzielt.

Die niedrigste, bereits wirksame Dosis in dem obenangegebenen Tierversuch ist beispielsweise

2 mg/kg oral
1 mg/kg intravenös
1 mg/kg subcutan.

Als allgemeiner Dosisbereich für die Wirkung (Tierversuch wie oben) kommt beispielsweise in Frage:

5 - 15 mg/kg oral
3 - 6 mg/kg intravenös
3 - 6 mg/kg subcutan.

Die Wirkungsrichtung der erfindungsgemäßen Verbindungen ist mit der Wirkung des bekannten Arzneimittels Tamoxifen vergleichbar.

Indikationen, für die die erfindungsgemäßen Verbindungen in Betracht kommen können: Mammacarcinom sowie sämtliche östrogenabhängige Tumore (zum Beispiel Endometriumcarcinom), Prostatacarcinom, benigne Prostatahyperplasie.

Die pharmazeutischen Zubereitungen enthalten im allgemeinen zwischen 25 bis 100, vorzugsweise 40 bis 60 mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: ölige oder alkoholische beziehungsweise wässrige Lösungen sowie Suspensionen und Emulsionen. Bevorzugte Anwendungsformen sind Tabletten, die zwischen 40 und 60 mg oder Lösungen, die zwischen 5 bis 10 % an aktiver Substanz enthalten.

Die Einzeldosis der erfindungsgemäßen aktiven Komponenten kann beispielsweise liegen

a) bei oralen Arzneiformen zwischen 40 und 60 mg, vorzugsweise 50 mg,
b) bei parenteralen Arzneiformen (zum Beispiel intravenös, intramuskulär) zwischen 20 bis 30 mg, vorzugsweise 25 mg,
c) bei Arzeneiformen zur lokalen Applikation auf die Haut und Schleimhäute (zum Beispiel in Form von Lösungen, Lotionen, Emulsionen, Salben und so weiter) zwischen 5 bis 10 %, vorzugsweise 7,5 %.

(Die Dosen sind jeweils bezogen auf die freie Base)

Beispielsweise können 3-mal täglich 1 bis 2 Tabletten mit einem Gehalt von 40 bis 60 mg wirksamer Substanz oder zum Beispiel bei intravenöser Injektion 1 - bis - 3 mal täglich eine Ampulle von 10 bis 30 ml Inhalt mit 20 bis 30 mg Substanz empfohlen werden. Bei oraler

Verabreichung ist die minimale tägliche Dosis beispielsweise 50 mg; die maximale tägliche Dosis bei oraler Verabreichung soll nicht über 400 mg liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med., 57 (1944), 261) liegt beispielsweise bei oraler Applikation oberhalb 2000 mg/kg.

Vorzugsweise erfolgt die Applikation der erfindungsgemäßen Verbindungen oral und intravenös oder auch intramuskulär oder subcutan.

**Beispiel 1**

2,3-Bis-(2-fluor-4-hydroxyphenyl)-2,3-dimethyl-butan

Eine Lösung von 3,34 g (0,01 Mol) 2,3-Bis(2-fluor-4-metoxy-phenyl)-2,3-dimethyl-butan in 250 ml trockenem Methylendichlorid wird auf -60° C gekühlt. In Stickstoffatmosphäre werden zu dieser Lösung unter Rühren 7,52 g (0,03 Mol) Bortribromid zugegeben. Nach 30 Minuten wird die Gefriermischung entfernt und die Reaktionsmischung 4 Stunden bei Raumtemperatur gehalten. Dann werden 50 $cm^3$ Methanol zugesetzt, mit 2 n NaOH geschüttelt und die wässrige Phase nach Neutralisieren mit 3 n HCl mit Diethylether extrahiert. Der nach Entfernen des Ethers erhaltene Rückstand wird mehrmals aus Benzol umkristallisiert.

F. 168 - 169° C
(Ausbeute: 88 %)

Herstellung der Ausgangssubstanz:

17,75 g (0,125 Mol) Methyljodid werden in Diethylether gelöst und tropfenweise unter Rühren zu 3,04 g Magnesiumspänen in 15 ml trockenem Ether gegeben. Die Mischung wird 30 Minuten unter Rückfluß erhitzt und anschließend hierzu unter Rühren tropfenweise eine Lösung von 16,82 g (0,1 Mol) 2-Fluor-4-methoxy-acetophenon in Diethylether gegeben. Nach 2 Stunden Erhitzen unter Rückfluß wird die Reaktionsmischung abgekühlt, auf Eis gegossen und der entsprechende Niederschlag durch Zusatz von wässriger $NH_4Cl$-Lösung aufgelöst. Die Etherphase wird abgetrennt, die wässrige Phase mit Ether extrahiert, die vereinigten Etherphasen mit wässrigen Lösungen von $NaHSO_3$, $NaHCO_3$ und anschließend mit reinem Wasser gewaschen, über $Na_2SO_4$ getrocknet, der Ether entfernt. Destillation des zurückbleibenden Öls unter Hochvakuum liefert 14,9 g 2-(2-Fluor-4-methoxy-phenyl)-2-propanol.

4,63 g (0,03 Mol) Titantrichlorid werden unter Stickstoff in 150 $cm^3$ trockenem Dimethoxyethan (glyme) suspendiert und hierzu 0,38 g (0,01 Mol) $LiAlH_4$ schnell unter Eiskühlung zugegeben. Die so erhaltene dunkle Suspension wird 10 Minuten gerührt. Dann werden 1,84 g (0,01 Mol) 2-(2-Fluor-4-methoxy-phenyl)-2-propanol, gelöst in 10 $cm^3$ trockenem Dimethoxyethan tropfenweise unter Rühren zugefügt und die Mischung 16 Stunden unter Rückfluß erhitzt. Dann läßt man abkühlen, zersetzt mit 2 n HCl, verdünnt mit Wasser und extrahiert mit Ether. Die Etherextrakte werden gewaschen ($NaHCO_3$, $H_2O$) und über $MgSO_4$ getrocknet. Nach Entfernen des Ethers wird das erhaltene 2,3-Bis-(2-fluor-4-methoxy-phenyl)-2,3-dimethyl-butan aus Toluol/Ligroin umkristallisiert.

**Beispiel 2**

2,3-Bis(2-fluor-4-acetoxy-phenyl)-2,3-dimethyl-butan

Zu einer eisgekühlten Lösung von 2,3-Bis(2-fluor-4-hydroxy-phenyl)-2,3-dimethyl-butan (306 mg; 1 mmol) in 30 ml Pyridin tropft man unter Rühren Essigsäureanhydrid (306 mg; 3 mmol) zu. Die Reaktionsmischung wird 3 Stunden zum Sieden erhitzt und nach dem Abkühlen mit Eis/$H_2O$ hydrolisiert. Nach Extraktion mit $CH_2Cl_2$ wäscht man die vereinigten organischen Extrakte nacheinander mehrmals mit 1 n HCl, gesättigter $NaHCO_3$-Lösung und $H_2O$ und trocknet über $MgSO_4$. Nach dem Entfernen des Lösungsmittels im Vakuum kristallisiert man aus Ethanol um.

Farblose Kristalle; F. 160 - 161° C (Ausbeute: 84 %)

**Beispiel 3**

2,3-Bis(2-fluor-4-dichloracetoxy-phenyl)-2,3-dimethylbutan

Zu einer vorgelegten Mischung aus 2,3-Bis(2-fluor-4-hydroxy-phenyl)-2,3-dimethyl-butan (1,53 g; 5 mmol) und Dichloracetanhydrid (6,0 g, 25 mmol) tropft man unter Rühren und Kühlung 15 ml trockenes Pyridin zu. Nach 30 Minuten Rühren des Reaktionsansatzes bei Raumtemperatur werden ca. 150 ml 50 %-iges wässriges Ethanol zugegeben; der Niederschlag wird abgesaugt und mit kaltem 50 %-igem wässrigem Ethanol mehrmals gewaschen. Danach kristallisiert man das Produkt vorsichtig aus Ethanol um, indem man allzu starkes Erhitzen vermeidet.

Farblose Kristalle; F. 125,5 - 126,5° C (Ausbeute: 40 %).

**Beispiel 4**

2,3-Bis(-2-fluor-4-carbamoyloxy-phenyl)-2,3-dimethyl-butan

Zu einer Lösung von 2,3-Bis(2-fluor-4-hydroxy-phenyl)-2,3-dimethyl-butan (1,53 g; 5 mmol) in 40 ml absolutem Aceton gibt man unter Eiskühlung Bromcyan (1,27 g; 12 mmol) zu. Unter starkem Rühren wird Triethylamin (1,21 g; 12 mmol) zugetropft, wobei die Temperatur 10° C nicht

überschreiten sollte. Nach 10 Minuten Rühren bei Raumtemperatur nutscht man das ausgefallene Triethylaminhydrobromid ab und wäscht 3-mal mit je 20 ml Aceton nach. Nach Entfernen des Lösungsmittels im Vakuum kristallisiert man das erhaltene Zwischenprodukt (Cyanat) aus Aceton um. Man versetzt das Cyanat zur Hydrolyse mit 30 ml halbkonzentrierter HCl und 2 ml konzentrierter $H_2SO_4$ und erhitzt die Mischung 0,5 - 1 Stunde, bis eine leichte Rosafärbung der Lösung eintritt. Nach schnellem Abkühlen saugt man das Rohprodukt ab und wäscht mit viel $H_2O$ nach. Das Carbamat wird aus Ethanol/$H_2O$ umkristallisiert.

Farblose Kristalle; F. 185,5 - 187°C (Ausbeute: 85 %, bezogen auf das Ausgangsphenol)

**Beispiel 5**

2,3-Bis(2-fluor-4-phosphato-phenyl)-2,3-dimethyl-butan

Ein Gemisch von 2,3-Bis(2-fluor-4-hydroxy-phenyl)-2,3-dimethyl-butan (3,06 g; 0,01 Mol), $POCl_3$ (7,75 g; 0,05 Mol), 2 ml Pyridin und 0,1 ml $H_2O$ wird 5 Stunden zum Sieden erhitzt. Überschüssiges $POCl_3$ wird im Vakuum vollständig entfernt. Man legt 20 ml $H_2O$ und 15 g Eis vor und rührt den Phosphorylierungsansatz heiß ein. Nach 1,5 Stunden Ausrühren läßt man den Niederschlag über Nacht absitzen und nutscht das Rohprodukt ab. Das noch feuchte Produkt wird in ca. 15 ml $H_2O$ suspendiert und 1 Stunde bei 20°C gerührt. Nach Aufheizen auf 70°C saugt man heiß ab. Eine erneute Suspension des Produkts in 5 ml $H_2O$ stellt man mit 4 n NaOH auf pH 7,8 ein, wobei die Temperatur maximal 40°C betragen darf. Die erhaltene Lösung wird filtriert und zu dem Filtrat werden innerhalb von 5 Minuten 15 ml einer auf 80°C erhitzten 5 n HCl zugegeben. Das so erhaltene Rohprodukt wird scharf abgesaugt und im Vakuum bei 50°C getrocknet. Nach Umfällen der Verbindung aus Methanol/Ether wird aus $H_2O$ umkristallisiert.

Farblose Kristalle; F. 191 - 193°C (Ausbeute: 42 %)

**Beispiel für pharmazeutische Zubereitungen**

**Beispiel für Kapseln**

10 g 2,3-Bis(2-fluor-4-hydroxy-phenyl)-2,3-dimethylbutan (mikronisiert), 106,7 g Calciumhydrogenphosphat (Ph.Eur.III) werden durch ein Sieb (1 mm Maschenweite) gegeben und gemischt. Diese Mischung wird mit einer Lösung aus 2,3 g Gelatine (DAB 8) und 1,0 g Polysorbat (80 Ph.Eur.III) in 20,7 g Wasser angefeuchtet, durch ein Sieb der Maschenweite 2 mm granuliert und bei 40°C getrocknet.

Dieses Granulat wird mit 20 g Maisstärke (Ph.Eur.III) über ein 0,8 mm Sieb gegeben und homogenisiert. Auf einer geeigneten Kapselmaschine füllt man diese Masse zu 140 mg in Hartgelatinekapseln der Größe 3. Eine Kapsel enthält 10 mg Wirkstoff.

**Beispiel für Tabletten**

10 g 2,3-Bis (2-fluor-4-carbamoyloxy-phenyl) -2,3-dimethyl-butan (mikronisiert) und 107,6 g Calciumhydrogenphoshat (Ph.Eur.III) werden gemischt und mit einer Lösung aus 2,3 g Gelatine (DAB 8) und 0,1 g Polysorbat (80 Ph.Eur.III) in 20,6 g Wasser angefeuchtet. Diese Masse wird durch ein 2 mm Sieb granuliert und bei 40°C getrocknet. Anschließend gibt man dieses Granulat, 35 g Maisstärke (Ph.Eur.III) und 5 g Talkum (Ph.Eur.III) durch ein Sieb (0,8 mm Maschenweite) und homogenisiert.

In üblicher Weise wird diese Masse auf einer geeigneten Maschine zu Tabletten von 160 mg Gewicht und einem Durchmesser von 7 mm gepreßt.

1 Tablette enthält 10 mg Wirkstoff.

DAB 8: Deutsches Arzneibuch, 8. Ausgabe

Ph.Eur.: Europäisches Arzneibuch, Band I, II oder III

**Beispiel für Injektionslösung**

In 1,8 Liter Wasser für Injektionszwecke werden 18 g Natriumchlorid zur parenteralen Anwendung (Ph.Eur.I) und 5 g 2,3-Bis(2-fluor-4-phosphato-phenyl)-2,3-dimetyl-butan unter Stickstoffbegasung gelöst und mit Wasser für Injektionszwecke auf 2 Liter aufgefüllt. Unter aseptischen Bedingungen wird nun über ein Membranfilter von 0,2 µm Porenweite sterilfiltriert. Schließlich füllt man diese Lösung unter aseptischen Bedingungen unter Stickstoffbegasung zu 2,15 ml in sterile 2 ml-Ampullen.

1 Ampulle enthält 5 mg Wirkstoff.

**Patentansprüche** für die Vertragsstaaten: BE, CH, DE, FR, GB, IT, LI, LU, NL, SE

1. Verbindungen der Formel

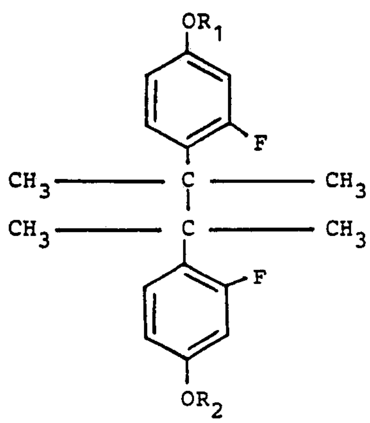

I

worin die Reste $R_1$ und $R_2$ gleich oder verschieden sind und Wasserstoff, eine Aminocarbonylgrruppe, eine $C_1$-$C_6$-Alkylaminocarbonylgruppe, eine Di-$C_1$-$C_6$-alkylaminocarbonylgruppe, die Gruppe $PO(OH)_2$, eine $C_2$-$C_8$-Alkanoylgruppe, eine $C_2$-$C_8$-Halogen-alkanoylgruppe oder eine $C_3$-$C_8$-Alkenoylgruppe bedeuten.

2. Verfahren zur Herstellung von Verbindungen der Formel I

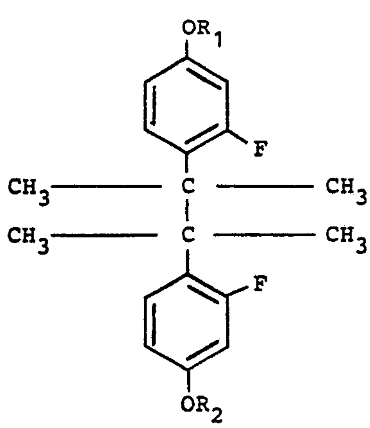

I

worin die Reste $R_1$ und $R_2$ gleich oder verschieden sind und Wasserstoff, eine Aminocarbonylgruppe, $C_1$-$C_6$-Alkylaminocarbonylgruppe, Di-$C_1$-$C_6$-alkylaminocarbonylgruppe, die Gruppe $PO(OH)_2$, eine $C_2$-$C_8$-Alkanoylgruppe, eine $C_2$-$C_8$-Halogen-alkanoylgruppe oder eine $C_3$-$C_8$-Alkenoylgruppe bedeuten,

dadurch gekennzeichnet,

daß man

in einer Verbindung der Formel

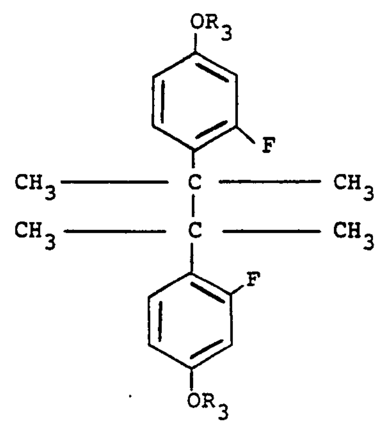

II

worin $R_3$ eine $C_1$-$C_4$-Alkylgruppe ist, diese beiden Alkylgruppen abspaltet und gegebenenfalls die erhaltene Verbindung der Formel I, worin beide Reste $R_1$ und $R_2$ Wasserstoff sind, durch die Reste $R_1$ und $R_2$ acyliert, wobei die Reste $R_1$ und $R_2$ die angegebenen Beutungen außer Wasserstoff haben und gegebenenfalls in den erhaltenen Verbindungen vorhandene $C_3$-$C_8$-Halogenalkanoylgruppen durch Abspaltung von Halogenwasserstoff in $C_3$-$C_8$-Alkenoylgruppen überführt.

3. Verbindungen der Formel I zur Anwendung als therapeutische Wirkstoffe.

4. Arzneimittel, enthaltend eine Verbindung der allgemeinen Formel I neben üblichen Träger und/oder Verdünnungs- beziehungweise Hilfsstoffen.

5. Verfahren zur Herstellung eines Arzneimittels,

dadurch gekennzeichnet,

daß eine Verbindung der allgemeinen Formel I mit gebräuchlichen pharmazeutischen Trägerstoffen und/oder Verdünnungsmitteln beziehungsweise sonstigen Hilfsstoffen zu pharmazeutiscnen Zubereitungen verarbeitet beziehungweise in eine therapeutisch anwendbare Form gebracht wird.

6. Verwendung von Verbindungen- der allgemeinen Formel I zur Herstellung von Arzneimitteln.

**Patentansprüche** für den Vertragsstaat: AT

1. Verfahren zur Herstellung von Verbindungen der Formel I

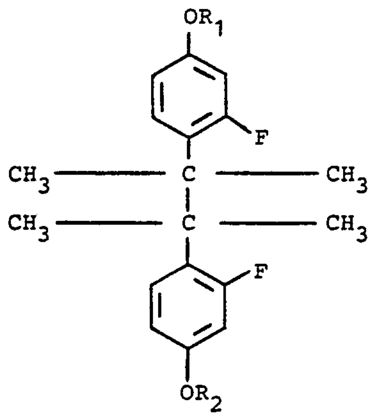

I

worin die Reste $R_1$ und $R_2$ gleich oder verschieden sind und Wasserstoff, eine Aminocarbonylgruppe, eine $C_1$-$C_6$-Alkylaminocarbonylgruppe, eine Di-$C_1$-$C_6$-alkylaminocarbonylgruppe, die Gruppe $PO(OH)_2$, eine $C_2$-$C_8$-Alkanoylgruppe, eine $C_2$-$C_8$-Halogen-alkanoylgruppe oder eine $C_3$-$C_8$-Alkenoylgruppe bedeuten,

dadurch gekennzeichnet,

daß man

in einer Verbindung der Formel

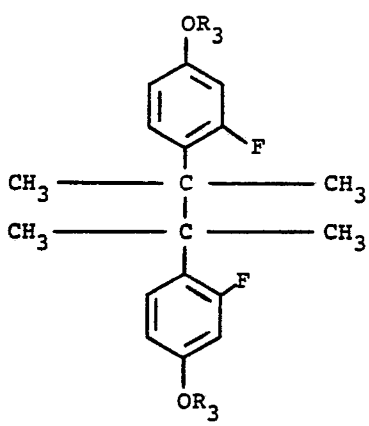

II

worin $R_3$ eine $C_1$-$C_4$-Alkylgruppe ist, diese beiden Alkylgruppen abspalten und gegebenenfalls die erhaltene Verbindung der Formel I, worin beide Reste $R_1$ und $R_2$ Wasserstoff sind, durch die Reste $R_1$ und $R_2$ acyliert, wobei die Reste $R_1$ und $R_2$ die angegebenen Bedeutungen außer Wasserstoff haben und gegebenenfalls in den erhaltenen Verbindungen vorhandene $C_3$-$C_8$-Halogenalkanoylgruppen durch Abspaltung von Halogenwasserstoff in $C_3$-$C_8$-Alkenoylgruppen überführt.

2. Verfahren zur Herstellung eines Arzneimittels, enthaltend als Wirkstoff mindestens eine Verbindung der allgemeinen Formel I, worin die Reste $R_1$ und $R_2$ die in Anspruch 1 angegebene Bedeutung haben,

dadurch gekennzeicnet,

daß mindestens eine Verbindung der Formel I mit gebräuchlichen pharmazeutischen Trägerstoffen und/oder Verdünnungsmittel beziehungsweise sonstigen Hilfsstoffen zu pharmazeutischen Zubereitungen verarbeitet beziehungsweise in eine therapeutisch anwendbare Form gebracht wird.

3. Verwendung von Verbindungen der allgemeinen Formel I, worin die Reste $R_1$ und $R_2$ die in Anspruch 1 angegebene Bedeutung haben, zur Herstellung von Arzneimitteln.

**Claims** for the contracting States; BE, CH, DE, FR, GB, IT, LI, LU, NL, SE

1. Compounds corresponding to the formula

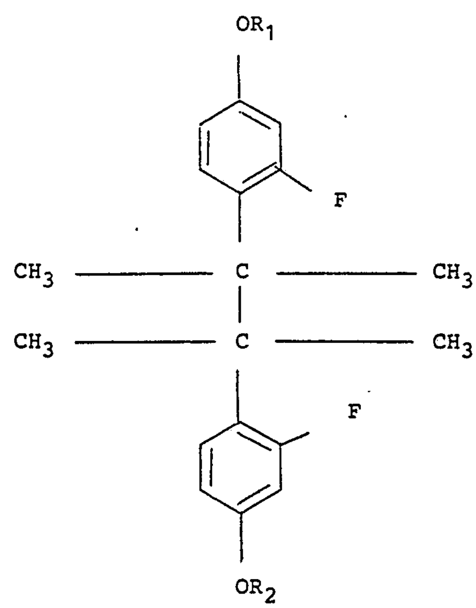

I

wherein the radicals $R_1$ and $R_2$ are the same or different and represent hydrogen, an aminocarbonyl group, a $C_1$-$C_6$ alkylaminocarbonyl group, a di-$C_1$-$C_6$ alkylaminocarbonyl group, the group $PO(OH)_2$, a $C_2$-$C_8$ alkanoyl group, a $C_2$-$C_8$ halogen-alkanoyl group or a $C_3$-$C_8$ alkenoyl group.

2. A process for the production of compounds

according to formula I

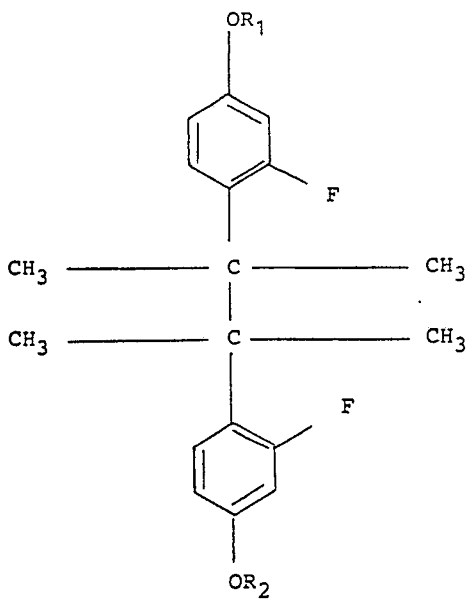

I

wherein the radicals $R_1$ and $R_2$ are the same or different and represent hydrogen, an aminocarbonyl group, a $C_1$-$C_6$ alkylaminocarbonyl group, a di-$C_1$-$C_6$ alkylaminocarbonyl group, the group $PO(OH)_2$, a $C_2$-$C_8$ alkanoyl group, a $C_2$-$C_8$ halogen-alkanoyl group or a $C_3$-$C_8$ alkenoyl group, characterised in that, in a compound corresponding to the formula

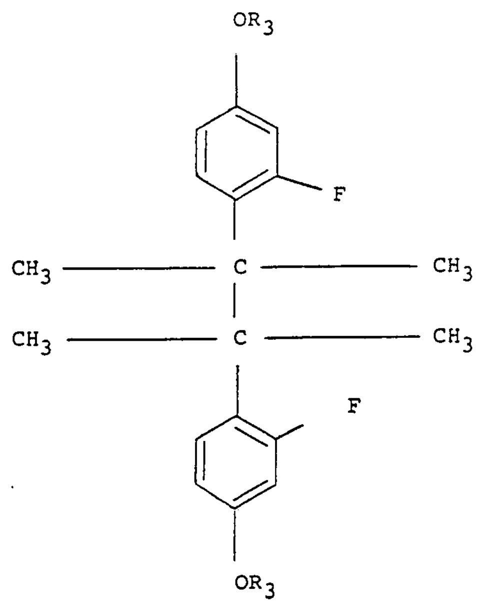

II

wherein $R_3$ is a $C_1$-$C_4$ alkyl group, these two alkyl groups are eliminated and the compound obtained according to formula I, in which both radicals $R_1$ and $R_2$ are hydrogen, is optionally acylated by the radicals $R_1$ and $R_2$ wherein the radicals $R_1$ and $R_2$ have the meanings given, except for hydrogen, and any $C_3$-$C_8$ halogenalkanoyl groups present in the compounds obtained are optionally converted into $C_3$-$C_8$ alkenoyl groups by elimination of hydrogen halide.

3. Compounds according to formula I for use as therapeutically active substances.

4. Pharmaceutical composition containing a compound according to general formula I in addition to conventional carriers and/or diluents and auxiliaries.

5. A process for the production of a pharmaceutical composition, characterised in that a compound according to general formula I is processed with conventional pharmaceutical carriers and/or diluents and other auxiliaries into pharmaceutical preparations or is brought into a therapeutically useful form.

6. Use of compounds according to general formula I for the production of pharmaceutical compositions.

**Claims** for the contracting State: AT

1. A process for the production of compounds according to formula I

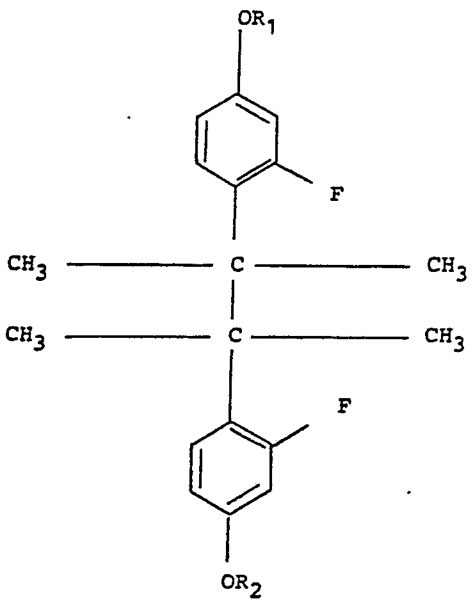

I

wherein the radicals $R_1$ and $R_2$ are the same or different and represent hydrogen, an aminocarbonyl group $C_1$-$C_6$ alkylaminocarbonyl group, a di-$C_1$-$C_6$ alkylaminocarbonyl group, the group $PO(OH)_2$, a $C_2$-$C_8$ alkanoyl group, a $C_2$-$C_8$ halogen-alkanoyl group or a $C_3$-$C_8$ alkenoyl group, characterised in that, in a compound corresponding to the formula

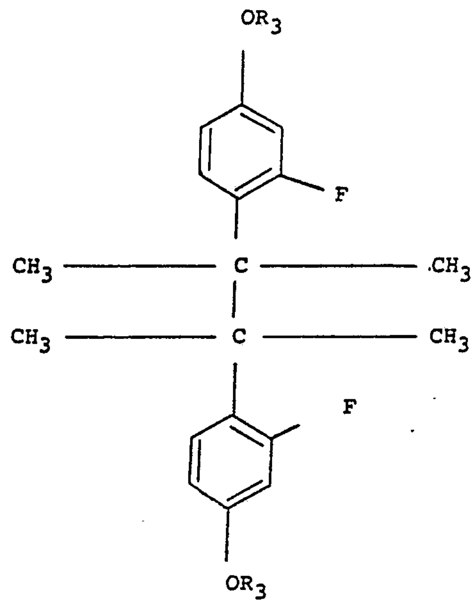

II

wherein $R_3$ is a $C_1$-$C_4$ alkyl group, these two alkyl groups are eliminated and the comopund obtained according to formula I, in which both radicals $R_1$ and $R_2$ are hydrogen, is optionally acylated by the radicals $R_1$ and $R_2$ wherein the radicals $R_1$ and $R_2$ have the meanings given, except for hydrogen, and any $C_3$-$C_8$ halogenalkanoyl groups present in the compounds obtained are optionally converted into $C_3$-$C_8$ alkenoyl groups by elimination of hydrogen halide.

2. Process for the production of a pharmaceutical composition containing as active ingredient at least a compound according to general formula I, wherein $R_1$ and $R_2$ have the given meanings, characterized in that at least a compound of formula I is processed with conventional pharmaceutical carriers and/or diluents and other auxiliaries into pharmaceutical preparations or is brought into a therapeutically useful form.

3. Use of compounds according to general formula I wherein the residues $R_1$ and $R_2$ have the meanings given in claim 1 for the production of pharmaceutical compositions.

**Revendications** four les Etats contractants: BE, CH, DE, FR, GB, IT, LI, LU, NL, SE

1. Composés de formule

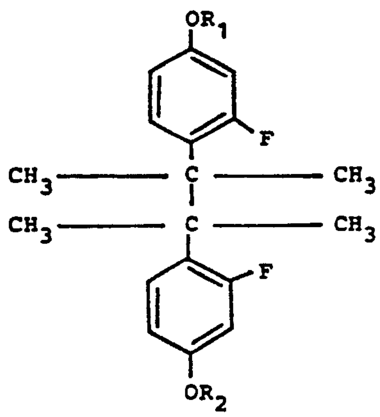

I

dans laquelle les radicaux $R_1$ et $R_2$ sont semblables ou différents et représentent chacun un atome d'hydrogène, un groupe aminocarbonyle, un groupe $C_1$-$C_6$-alkylaminocarbonyle, un groupe di-$C_1$-$C_6$-alkylaminocarbonyle, le groupe $PO(OH)_2$, un groupe alcanoyle en $C_2$-$C_6$, un groupe alcanoyle halogéné en $C_2$-$C_8$ un groupe alcénoyle en $C_3$-$C_8$.

2. Procédé pour la préparation de composés de formule I

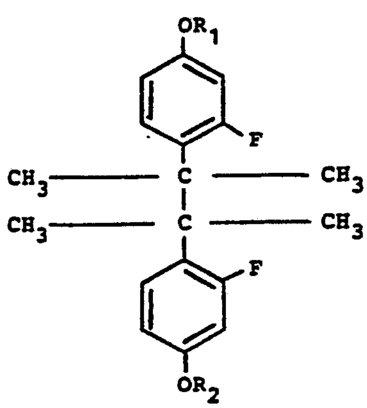

I

dans laquelle les radicaux $R_1$ et $R_2$ sont semblables ou différents et représentent chacun un atome d'hydrogène, un groupe aminocarbonyle, un groupe $C_1$-$C_6$-alkylaminocarbonyle, un groupe di-$C_1$-$C_6$-alkylaminocarbonyle, le groupe $PO(OH)_2$, un groupe alcanoyle en $C_2$-$C_6$ un groupe alcanoyle halogéné en $C_2$-$C_6$ ou un groupe alcénoyle en $C_3$-$C_8$, caractérisé en ce que dans un composé de formule

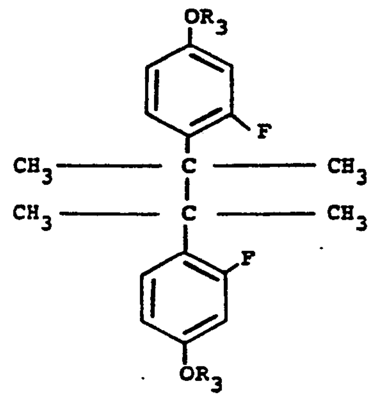

II

dans laquelle $R_3$ est un groupe alkyle en $C_1$-$C_4$ ces deux groupes alkyle sont éliminés et, le cas échéant, le composé obtenu de formule I, dans laquelle les deux radicaux $R_1$ et $R_2$ sont des atomes d'hydrogène, est acylé par les radicaux $R_1$ et $R_2$, les radicaux $R_1$ et $R_2$ ayant les signification déjà données en dehors de l'hydrogène, et les groupes alcanoyle halogéné en $C_3$-$C_8$ éventuellement présents dans les composés obtenus sont transformés en groupes alcénoyle en $C_3$-$C_8$ par déshydrohalogénation.

3. Composés de formule I, dans leur utilisation comme substances thérapeutiquement actives.

4. Médicament contenant un composé de formule générale I, outre des excipients et/ou diluants ou adjuvants usuels.

5. Procédé de préparation d'un medicament, caractérisé en ce qu'un composé de formule générale I est transformé en préparations pharmaceutiques ou est mis sous une forme utilisable en thérapeutique, avec des excipients et/ou diluants ou autres adjuvants pharmaceutiques usuels.

6. Utilisation de composée de formule générale I pour la préparation de médicaments.

**Revendications** four l'Etat contractant: AT

1. Procédé pour la préparation de composés de formule I

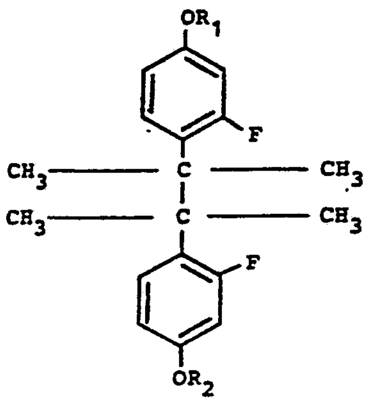

I

dans laquelle les radicaux $R_1$ et $R_2$ sont semblables ou différents et représentent chacun un atome d'hydrogène, un groupe aminocarbonyle, un groupe $C_1$-$C_6$-alkylaminocarbonyle, un groupe di-$C_1$-$C_6$-alkylaminocarbonyle, le groupe $PO(OH)_2$, un groupe alcanoyle en $C_2$-$C_8$, un groupe alcanoyle halogéné en $C_2$-$C_8$ ou un groupe alcénoyle en $C_3$-$C_8$, caractérisé en ce que dans un composé de formule

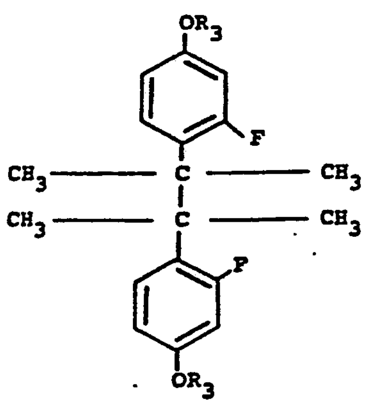

II

dans laquelle $R_3$ est un groupe alkyle en $C_1$-$C_4$, ces deux groupes alkyle sont éliminés et, le cas échéant, le composé obtenu de formule I, dans laquelle les deux radicaux $R_1$ et $R_2$ sont des atomes d'hydrogène, est acylé par les radicaux $R_1$ et $R_2$, les radicaux $R_1$ et $R_2$ ayant les signification déjà données en dehors de l'hydrogène, et les groupes alcanoyle halogéné en $C_3$-$C_8$ éventuellement présents dans les composés obtenus sont transformés en groupes alcénoyle en $C_3$-$C_8$ par déshydrohalogénation.

2. Procedé de préparation d'un médicament ayant comme substance active le moins un composé de formula général I dans laquelle les radicaux $R_1$ et $R_2$ ont les significations données dans revendication 1 caractérisé en ce qu'un composé de formule générale I est transformé en préparations pharmaceutiques ou est mis sous une forme utilisable en thérapeutique, avec des excipients et/ou diluants ou autres adjuvants pharmaceutiques usuels.

3. Utilisation de composés de formule générale I dans laquelle les radicaux $R_1$ et $R_2$ ont les significations données dans revendication 1 pour la préparation de médicaments.